# EUROPEAN PATENT APPLICATION

(11) **EP 3 120 812 A1**
(43) Date of publication of application: **25.01.2017**
(21) Application number: 16180861.3
(22) Date of filing: 22.07.2016
(51) Int. Cl.: A61F 2/34, A61F 2/46

(54) **DEVICE AND METHOD FOR GRIPPING AND POSITIONING PROSTHESIS PARTS**

(30) Priority: 23.07.2015 IT UB20152433
(71) Applicant: Amil S.r.l., 33034 Fagagna (IT)
(72) Inventor: LUALDI, Tommaso, 33034 Fagagna (UD) (IT)
(74) Representative: Petraz, Gilberto Luigi

(57) **Abstract**

Device (10) for gripping and positioning prosthesis parts, comprising: a gripping head (27) provided with one or more air passage channels (29) and configured to be inserted inside a seating (28) of a prosthesis part (11); a depressor unit (19) selectively drivable to determine a condition of depression or vacuum that is transmitted to the channels (29) of the gripping head (27) so that the head (27) securely holds the prosthesis part (11).

## Description

### FIELD OF THE INVENTION

The present invention concerns the field of application of orthopedic prostheses and in particular a device and a method, in particular a manual device and method, for gripping and positioning prosthesis parts, for example prosthesis inserts, which form part of orthopedic prostheses.

### BACKGROUND OF THE INVENTION

It is known that during surgical operations for the application of orthopedic prostheses, it is provided to apply prosthesis parts such as prosthetic inserts with shapes and sizes that vary according to the zone of the body where the prosthesis is required. One common type of installation of orthopedic prostheses is, for example, the application of hip prostheses. In this application, a cup-shaped acetabular prosthetic insert is implanted in the acetabular cavity of the pelvis, then a spherical head of an articulation insert is inserted, generally in jointed fashion, on the prosthetic insert. The orthopedic prosthesis is then completed in this case by a femoral rod that is implanted on one side in the patient's femur and on the other side in a seating of the spherical head, opposite the insertion seating in the acetabular insert.

The materials used for making prosthetic inserts and the corresponding articulation heads have to be biostable and bioinert and to this purpose said prosthetic inserts and articulation heads can be made for example of biocompatible ceramic materials, for example alumina.

One known instrument for inserting prosthetic inserts is described for example in US 2009/0281550 A1. Document DE19722923 A1 describes a known method for inserting ceramic caps into metal shells of articular prostheses. Document EP 1 698 306 A1 describes another known instrument for inserting acetabular prostheses. Document FR 2 809 305 A1 also describes a surgical instrument used in a known manner in the application of hip prostheses.

A first problem found in the application of orthopedic prostheses is that during the positioning of the prosthetic inserts, contact between the instruments used and the material of which they consist, for example ceramic material, tends to cause the release of particles that can cause imperfect coupling with the articulation head of the prosthesis, with consequent problems for the patient, which can also entail the failure of the prosthetic installation.

Another problem of known prosthetic applications, in particular in the application of hip prostheses, is connected to the fact that the release of particles from the prosthetic inserts can cause so-called "squeaking" phenomena, which can be very annoying for the patient and which clearly cause an imperfect and insecure installation of the orthopedic prosthesis.

Other problems relating to instruments and devices for inserting prosthetic inserts are that they lack linearity and precision in the operations to grip and release the prosthetic inserts.

It is therefore desirable that the installation of the prosthetic insert in the required zone of the patient's skeleton is performed in the most precise manner possible, safe and harmless for the patient, so as to then guarantee an effective and precise coupling of the articulation head of the prosthesis and preventing the loss of particles from the prosthetic insert.

There is therefore a need to perfect a device and method for gripping and positioning prosthesis parts that can overcome at least one of the disadvantages of the state of the art.

One purpose of the present invention is therefore to obtain a manual device for gripping and positioning prosthesis parts, for example prosthetic inserts, that allows a precise and safe installation of an orthopedic prosthesis, limiting to the utmost possible problems for the patient.

Another purpose of the present invention is to obtain a manual device for gripping and positioning prosthesis parts, for example prosthetic inserts, which thanks to its extremely simple and linear functioning allows to eliminate the loss of particles from the prosthetic insert, and thus prevent them from depositing in the zone of the skeleton where the orthopedic prosthesis is to be installed.

Another purpose of the present invention is to obtain a manual device for gripping and positioning prosthesis parts, for example prosthetic inserts, which can be completely dis-assembled so that every single part of it can be cleaned and sterilized.

Another purpose of the present invention is to obtain a manual device for gripping and positioning prosthesis parts which comprises effective gripping means and linear, immediate release means, and which do not entail sudden movements.

Another purpose of the present invention is to obtain a manual device for gripping and positioning prosthesis parts in which the gripping and release movements can be performed using simple and linear gestures, so as to always guarantee precision and accuracy in operations.

Another purpose of the present invention is to perfect a method for gripping and positioning prosthesis parts, for example prosthetic inserts, which is rapid, effective, which allows gripping and release to be performed simply, linearly and precisely, and which also allows to keep unchanged the quality of the prosthetic insert and hence of the orthopedic prosthesis obtained.

The Applicant has devised, tested and embodied the present invention to overcome the shortcomings of the state of the art and to obtain these and other purposes and advantages.

Other limitations and disadvantages of conventional solutions and technologies will be clear to a person of skill after reading the remaining part of the present description with reference to the drawings and the description of the embodiments that follow, although it is clear that the description of the state of the art connected to the present description must not be considered an admission that what is described here is already known from the state of the prior art.

### SUMMARY OF THE INVENTION

The present invention is set forth and characterized in the independent claims, while the dependent claims describe other characteristics of the invention or variants to the main inventive idea.

In accordance with the above purposes, a device for gripping and positioning prosthesis parts according to the present invention is characterized by the fact that it comprises a gripping head provided with one or more air passage channels and configured to be inserted inside a seating of a prosthesis part; a depressor unit configured to selectively generate, when driven, a condition of depression or vacuum that is transmitted to the channels of the gripping head in communication with said chamber, so that the head securely holds the prosthesis part. The depressor unit is provided with a chamber in which a piston is housed and which is in communication with the air passage channels; and at least a command lever connected to the piston and configured to translate the piston from at least a first inactive position, in which the chamber is substantially at atmospheric pressure, to at least a second active position in which, in the chamber, the depression to hold the prosthesis part is generated by means of the translation of the piston. The piston comprises a longitudinal channel in communication on one side with the chamber of the depressor unit and on the other side with a longitudinal hole made in the command lever. The command lever can comprise a stopper configured to put the longitudinal hole of the lever into communication with the outside environment and with the chamber of the depressor unit. The stopper of the command lever is associated with elastic return means housed inside the command lever and configured to keep the stopper raised and in a closed position, and with a button to automatically release the head from the prosthesis part.

Advantageously, using the device according to the invention for gripping and positioning prosthesis parts, for example prosthetic inserts, it is possible to position a prosthetic insert effectively and precisely, eliminating possible losses of material from the prosthetic insert. The gripping and positioning device is also simple and linear to use and can be completely dis-assembled, to clean and sterilize it completely and efficiently. The gripping device also allows linear, simple and precise gripping and release operations, thanks to the button associated with the lever.

According to another aspect of the invention, the device comprises a support rod in which a longitudinal channel is made in communication on one side with the air passage channels in the gripping head and on the other side with the chamber of the depressor unit.

Preferably, the air passage channels made in the gripping head are angularly offset, so as to create an even more uniform grip of the prosthesis part.

The depressor unit can comprise a handle element in which at least a first clamping seating is made to clamp the command lever in the first inactive position of the piston and at least a second clamping seating to clamp the command lever in the second active position of the piston.

According to another characteristic of the invention, the piston is associated with elastic return means that tend to make it assume the second active position.

The handle element can also comprise a plurality of alignment rods.

The rod can be inserted in a support provided, on the side opposite the side where the rod is inserted, with a flange for positioning the prosthesis part.

Preferably, the positioning flange comprises pins configured to be inserted in corresponding seatings of the prosthetic insert.

The present invention also concerns a method for gripping and positioning prosthetic inserts, characterized in that it comprises the following steps: inserting a prosthesis part on a gripping head provided with one or more air passage channels connected to a chamber of a depressor unit configured to make a vacuum condition in the chamber; driving the depressor unit so that in the air passage channels in contact with the prosthesis part a depression is created and so that the gripping head securely holds the prosthesis part.

According to a characteristic aspect, the present method comprises an automatic release step of the gripping head of the prosthesis part, executed by disposing the chamber of the depressor unit at atmospheric pressure by means of a button associated with a multifunction lever with which the gripping operation is also performed.

These and other aspects, characteristics and advantages of the present disclosure will be better understood with reference to the following description, drawings and attached claims. The drawings, which are integrated and form part of the present description, show some embodiments of the present invention, and together with the description, are intended to describe the principles of the disclosure.

The various aspects and characteristics described in the present description can be applied individually where possible. These individual aspects, for example aspects and characteristics described in the attached dependent claims, can be the object of divisional applications.

It is understood that any aspect or characteristic that is discovered, during the patenting process, to be already known, shall not be claimed and shall be the object of a disclaimer.

### BRIEF DESCRIPTION OF THE DRAWINGS

These and other characteristics of the present invention will become apparent from the following description of some embodiments, given as a non-restrictive example with reference to the attached drawings wherein:
- fig. 1 shows a first perspective and exploded view of a manual device for gripping and positioning prosthetic inserts according to the present invention;
- fig. 2 shows a second perspective view of the present positioning device when assembled;
- fig. 3a shows a first partial view, in longitudinal section, of the present device in a first inactive position, being moved toward a prosthetic insert;
- fig. 3b shows a second partial view, in longitudinal section, of the present device in a second intermediate position, gripping the prosthetic insert;
- fig. 3c shows a third partial view, in longitudinal section, of the present device in a third active position, holding the prosthetic insert.

To facilitate comprehension, the same reference numbers have been used, where possible, to identify identical common elements in the drawings. It is understood that elements and characteristics of one embodiment can conveniently be incorporated into other embodiments without further clarifications.

### DETAILED DESCRIPTION OF SOME EMBODIMENTS

We shall now refer in detail to the various embodiments of the present invention, of which one or more examples are shown in the attached drawing. Each example is supplied by way of illustration of the invention and shall not be understood as a limitation thereof. For example, the characteristics shown or described insomuch as they are part of one embodiment can be adopted on, or in association with, other embodiments to produce another embodiment. It is understood that the present invention shall include all such modifications and variants.

Before describing these embodiments, we must also clarify that the present description is not limited in its application to details of the construction and disposition of the components as described in the following description using the attached drawings. The present description can provide other embodiments and can be obtained or executed in various other ways. We must also clarify that the phraseology and terminology used here is for the purposes of description only, and cannot be considered as limitative.

By way of example the following detailed description of some embodiments concerns a prosthetic insert, but it is clear that the device and method according to the present invention can be applied for the above purposes and obtaining the same advantages for other elements or prosthesis parts as well.

With reference to the attached drawings, 10 denotes a device according to the present invention for gripping and positioning a prosthesis part, for example prosthetic inserts 11, such as a hemi-spherical insert, for example an acetabular insert or other.

The positioning device 10 comprises a rod 12 with a longitudinal channel 13 for the air to pass through. The rod 12 comprises a first end 14 configured to be inserted in embedded fashion in a support 15, with a truncated cone shape, and a second threaded end 16 configured to screw into a female screw 18 of a flange 17 made at the base of a depressor unit 19 configured to obtain a condition of depression or vacuum.

The truncated cone support 15, in correspondence with the part with the biggest section, has an annular housing 20 configured to receive a flange 21 on which a plurality of pins 22 are made. The pins 22 can be engaged in corresponding holes 23 made in the prosthetic insert 11, so as to guarantee a correct positioning thereof and to prevent unwanted reciprocal rotations of the rod 12 and prosthetic insert 11. Inside the support 15 a female screw 24 is also made, in a longitudinal direction, and configured to receive a corresponding threaded pin 25 bearing longitudinally a through hole 26.

The threaded pin 25 is part of a head 27 for holding the prosthetic insert 11 I configured to be inserted inside a seating 28 made in the prosthetic insert 11. In this case, the seating 28 of the prosthetic insert 11 is semi-spherical in shape, therefore the head 27 also has a semi-spherical shape. A plurality of channels 29 are made in the head 27 and, on the side facing toward the prosthetic insert 11, communicate with the outside and on the other side communicate with the through hole 26 made in the threaded pin 25. An annular groove 30 is also made in the head 27, for housing a sealing packing 31, for example an 0-ring.

A plurality of female screws 32, for example three, are made on the flange 17 of the depressor unit 19, having a certain inclination and provided with a thread configured to engage with the corresponding threaded end 33 of a plurality of rods 34 for aligning the positioning device 10.

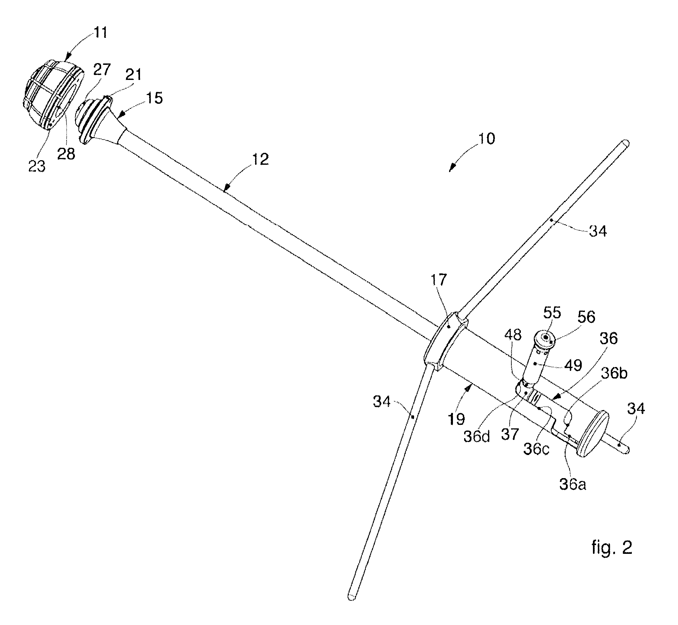

The depressor unit 19 comprises a handle element 35 which on one side is closed by the flange 17 provided with the female screw 18 and on the other side is open toward the outside and can be closed by a sealing stopper 59, for example made of rubber or other soft elastic material. The handle element 35 is cylindrical in shape for example. On the surface of the handle element 35 a groove 36 is made, for bayonet-type attachment, with four segments 36a, 36b, 36c and 36d (fig. 2): a first longitudinal segment 36a, a second transverse segment 36b, a third longitudinal segment 36c and a fourth transverse segment 36d. Inside the handle element 35 a chamber 58 is made in which a piston 37 is housed, bearing longitudinally a through hole 38 and at the ends two female screws 39 and 40. A first screw 41 to close the piston 37 is screwed into the first female screw 39, while a second screw 42, in which a longitudinal through hole 43 is made, is screwed into the second female screw 40. Between the second screw 42 and the end of the piston 37 a sealing packing 44 is housed, for example an 0-ring.

An elastic return element 45, for example a spring, is housed in a seating 46 made at the end of the chamber 58 of the handle element 35, opposite the end where the stopper 59 is provided. One end of the elastic element 45 abuts against the surface of the second screw 42, while the other end abuts on the bottom of the seating 46.

On the external surface of the handle element 35 and near the end where the first screw 41 is housed, that is, the end farthest from the elastic return element 45 and hence from the holding head 27 of the insert 11, a housing seating 47 is made, for housing an end 48 of a command lever 49 of the depressor unit 19 to generate a vacuum. The end 48 of the lever 49 can be inserted in embedded fashion into the seating 47, or it can be provided that the end has a thread configured to engage with a corresponding female screw made in the seating 47. The lever 49 comprises internally a longitudinal channel 50 for the air to pass through, which on one side communicates with the outside by means of radial holes 51 made on the lever 49 and situated near the end of the lever 49 and, on the other side, communicates with the female screw 39. The female screw 39 also communicates with the through hole 38 made in the piston 37.

Inside the longitudinal channel 50 made in the lever 49, an elastic return element 52 is longitudinally housed, which abuts at one end against a stopper 53 provided laterally with longitudinal gaps 54 for the air to pass through. The stopper 53 is positioned on a rod 55, on the top of which a button 56 is screwed. Between the stopper 53 and the end part of the lever 49 a sealing packing 57 is housed, for example an 0-ring.

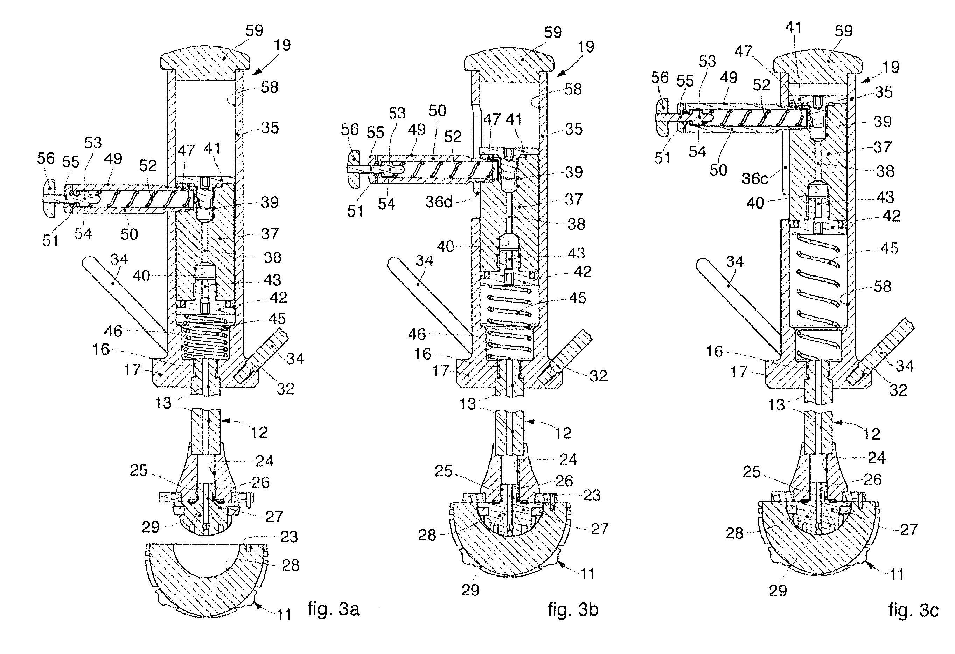

The functioning of the device 10 will be clear from the following description. In a first inactive position shown in figs. 2 and 3a for example, the device 10 is moved near to the prosthetic insert 11, gripping the handle element 35. In this first inactive position, the lever 49 of the depressor unit 19 is at the end of the transverse segment 36d of the groove 36 made on the handle element 35. The piston 37 housed inside the chamber 58 of the handle element 35 is therefore in an inactive position and completely lowered, then inserted completely into the chamber 58, with the elastic element 45 compressed. The transverse segment 36d of the groove 36 allows to keep the position of the piston 37 without exerting any force on the lever 49. In fact, the elastic return element 45, being compressed, would tend to return the piston 37 upward.

The head 27 is inserted inside the seating 28 of the prosthetic insert 11 (see fig 3b), so that the pins 22 of the flange 21 are inserted in the corresponding holes 23 of the prosthetic insert 11. The lever 49 is lifted or extracted so that from the transverse segment 36d of the groove 36 it passes to the longitudinal segment 36c. In this intermediate situation, the elastic element 45 continues thrusting the piston 37 toward the upper part of the handle element 35, so toward the stopper 59. The lifting or extraction of the piston 37 already determines a certain depression in the chamber 58 and the seating 46. The seating 46 made on the bottom of the chamber 58 communicates with the longitudinal channel 13 made in the rod 12, and hence with the female screw 24, the through hole 26 and finally with the channels 29 made in the head 27, so that ultimately the depression is transferred to the channels 29 of the gripping head 27.

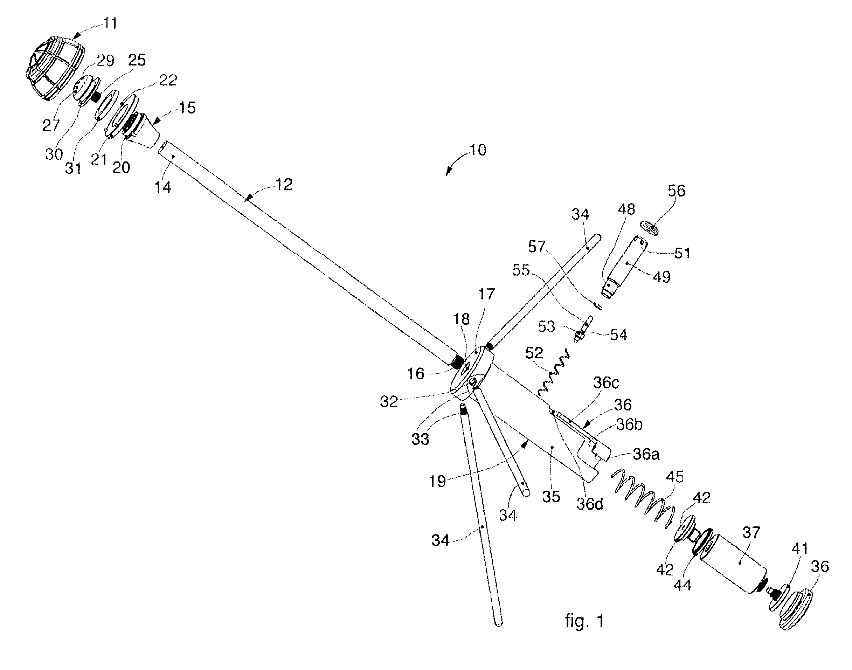

The depression or vacuum is increased by letting the lever 49 slide further (see fig. 3c), so that it travels the whole longitudinal segment 36c of the groove 36 and is positioned in the transverse segment 36b (fig. 1) of the groove 36. In this active position therefore, there will be a maximum suction of air by the piston 37 that is lifted and then extracted, until the situation shown in fig. 3c. In this step the elastic element 45 can be in an inactive condition. The housing of the lever 49 in the transverse segment 36b of the groove 36 allows to prevent accidental movements of the lever 49, which could even knock against the stopper 59 in an unwanted manner. Therefore, in the situation shown in fig. 3c, the holding head 27 and the prosthetic insert 11 are perfectly centered and attached to each other by means of the perfect adherence of the packing 31 and the flange 21.

The channels 29 made in the head 27 are angularly distanced from each other so as to guarantee a uniform distribution of the vacuum on the head 27 and hence a uniform gripping surface on the seating 28 of the prosthetic insert 11.

In this situation the prosthetic insert 11 can easily be taken to the right application position, gripping and possibly thrusting the handle element 35 in which the lever 49 is advantageously clamped in position in the transverse segment 36b of the groove 36. The manual positioning of the prosthetic insert 11 in the corresponding application seating can also be concluded by using alignment rods 34, which allow an even more precise positioning.

Once the prosthetic insert 11 has been put correctly into position, the positioning device 10 is separated. The separation is obtained simply and effectively by using the button 56, hence without having to again translate the lever 49 and by means of a small, controlled movement.

The button 56 is pressed against the action of the elastic element 52, which tends to keep it in the positions shown in the drawings. Lowering the button 56 causes a lowering of the stopper 53, and then a translation thereof toward the inside of the longitudinal channel 50, and then the air enters from the radial holes 51 and passes through the gaps 54 made on the periphery of the stopper 53. The air passes through the whole lever 49, the female screw 39, the through hole 38 of the piston 37, the through hole 43 and then arrives in the chamber 58. By means of this operation, in substance, the chamber 58 where the piston 37 slides is put at atmospheric pressure. By taking the chamber 58 to atmospheric pressure, in short, the channels 29 of the holding head 27 are also taken to atmospheric pressure, and therefore the effect of holding the head 27 on the prosthetic insert 11 stops, and this will be released in the desired position.

Once released, the button 56 returns to the position shown in the drawings thanks to the action of the elastic element 52, then the rod 55 and the stopper 53 provided with the sealing packing 57 again move to the closed position, thus preventing the entry of air from the radial holes 51.

Therefore, by means of a simple and linear gesture of pressing the button 56, the holding head 27 is separated from the prosthetic insert 11.

Once the manual positioning device 10 has been separated from the prosthetic insert 11, now in position, it is possible to return the piston 37 and the lever 49 again to the position shown in fig. 3a, so that the device is ready for new use.

The function of the last segment 36a of the groove 36 is to take the lever 49 to an inactive upper position by means of which it is possible to dis-assemble and clean the positioning device 10.

The device 10 for gripping and positioning prosthetic inserts is advantageously completely dis-assemblable, as shown in detail in the three-dimensional and exploded view in fig. 1, so that all the elements that make it up can be completely cleaned and sterilized.

It is clear that modifications and/or additions of parts may be made to the device and method for gripping and positioning prosthetic inserts as described heretofore, without departing from the field and scope of the present invention.

It is therefore clear that, although the present invention has been described with reference to some specific examples, a person of skill in the articulation shall certainly be able to achieve many other equivalent forms of device and method for gripping and positioning prosthetic inserts, having the characteristics as set forth in the claims and hence all coming within the field of protection defined thereby.

In the following claims, the sole purpose of the references in brackets is to facilitate reading: they must not be considered as restrictive factors with regard to the field of protection claimed in the specific claims.

## Claims

1. Device (10) for gripping and positioning prosthesis parts, comprising: a gripping head (27) provided with one or more air passage channels (29) and configured to be inserted inside a seating (28) of a prosthesis part (11); a depressor unit (19) selectively drivable to determine a condition of depression or vacuum that is transmitted to the channels (29) of the gripping head (27) so that said head (27) securely holds the prosthesis part (11), **characterized in that** said depressor unit (19) comprises:
- a chamber (58) in which a piston (37) is housed and which is in communication with said air passage channels (29);
- at least a command lever (49) connected to said piston (37) and configured to translate said piston (37) from at least a first inactive position, in which the chamber (58) is substantially at atmospheric pressure, to at least a second active position in which, in said chamber (58) and in said channels (29), said depression is generated by means of the translation of the piston (37);
- said piston (37) comprising a longitudinal hole (38) in communication on one side with said chamber (58) and on the other side with a longitudinal channel (50) made in the command lever (49),
- said command lever (49) comprising a stopper (53) configured to put said longitudinal channel (50) into communication with the outside environment and with said chamber (58) of the depressor unit (19),
- said stopper (53) of the command lever (49) being associated with elastic return means (52) housed inside said command lever (49) and configured to keep said stopper (53) raised and in a closed position, and also associated with a button (56) to automatically release the head (27) from the prosthesis part (11).

2. Device as in claim 1, **characterized in that** said depressor unit (19) comprises a handle element (35) in which at least a first clamping seating (36d) is made to clamp the command lever (49) in the first inactive position of the piston (37) and at least a second clamping seating (36b) to clamp the command lever (49) in the second active position of the piston (37).

3. Device as in claim 1 or 2, **characterized in that** said piston (37) is associated with elastic return means (45) that tend to make it assume said second active position.

4. Device as in any claim hereinbefore, **characterized in that** it comprises a support rod (12) in which a longitudinal channel (13) is made in communication on one side with said air passage channels (29) of the gripping head (27) and on the other side with said chamber (58) of the depressor unit (19).

5. Device as in any claim hereinbefore, **characterized in that** said rod (12) is inserted in a support (15) provided, on the opposite side to that where the rod (12) is inserted, with a positioning flange (21) of the prosthesis part (11).

6. Device as in claim 5, **characterized in that** said positioning flange (21) comprises pins (22) configured to be inserted into corresponding seatings (23) of the prosthesis part (11).

7. Method for gripping and positioning prosthetic inserts, **characterized in that** it comprises the following steps: inserting a prosthesis part (11) on a gripping head (27) provided with one or more air passage channels (29) connected to a chamber (58) of a depressor unit (19) configured to make a vacuum condition in said chamber (58); driving said depressor unit (19) so that in the air passage channels (29) in contact with the prosthesis part (11) a depression is created and so that the gripping head (27) securely holds the prosthesis part (11); an automatic release step of the gripping head (27) of the prosthesis part (11), executed by disposing the chamber (58) of the depressor unit (19) at atmospheric pressure by means of a button (56) associated with a multifunction lever (49) with which the gripping operation is also performed.
